# EUROPEAN PATENT APPLICATION

(11) **EP 2 854 054 A1**
(43) Date of publication of application: **01.04.2015**
(21) Application number: 14181943.3
(22) Date of filing: 22.08.2014
(51) Int. Cl.: G06F 19/00

(54) **Processing method, processing program, and infected animal electronic medical record device**

(30) Priority: 30.09.2013 JP 2013205537
(71) Applicant: FUJITSU LIMITED, Kawasaki-shi, Kanagawa 211-8588 (JP)
(72) Inventor: Sato, Masakazu, Kanagawa, 211-8588 (JP); Kimoto, Ai, Kanagawa, 211-8588 (JP)
(74) Representative: Hoffmann Eitle

(57) **Abstract**

An electronic medical record device (100) stores a medical condition or an observation about an infected animal as an item name, and displays whether the item name corresponds to completion or incompletion of treatment in a visually recognizable manner in a case of a follow-up examination. When selection of an item name has been made, the electronic medical record device (100) permits an input of treatment information corresponding to the medical condition or the observation about the infected animal, and stores the input treatment information in association with the selected item name.

## Description

### FIELD

The embodiments discussed herein is related to a processing method in an infected animal electronic medical record device, and the like.

### BACKGROUND

In a typical hospital that examines a human, a patient selects a diagnosis and treatment department according to an own medical condition, and is examined by a specialist in each diagnosis and treatment department. In contrast, an animal hospital is a general diagnosis and treatment department, and a doctor in the animal hospital may sometimes examine a plurality of causes of disease in one examination. In the following description, a cause of disease of an animal is described as problem, and a doctor of an animal hospital is simply described as doctor. Further, an animal to be treated may sometimes be described as infected animal.

When examining an animal, the doctor refers to a paper medical record created in the past, compares a problem that the animal to be examined suffered from in the past and a current medical condition of the animal to perform various determination related to treatment, and adds treatment information in the paper medical record.

For example, when the animal to be examined suffers from a disease similar to before, and treatment has not been completed, the doctor adds treatment information related to a course of the disease or a history of treatment content in an appropriate problem column in the paper medical record. Further, when the animal to be examined suffers from a disease that the animal has never suffered from before, the doctor prepares a new problem column in the paper medical record and adds the treatment information.

Patent Literature 1: Japanese Laid-open Patent Publication No. 11-45301

However, the above-described conventional technology has a problem that a problem that is an object to be input of treatment information is not easily recognized.

As described above, since an animal hospital is a general diagnosis and treatment department, pieces of information related to a plurality of types of problems are mixed in a paper medical record of an animal, and a problem that is an object to be input of the treatment information cannot be easily recognized.

Accordingly, it is an object in one aspect of an embodiment of the invention to provide a processing method in an infected animal electronic medical record device capable of displaying a screen that enables a user to easily recognize a problem that is an object to be input of treatment information, and a processing program, and an infected animal electronic medical record device.

### SUMMARY

According to an aspect of an embodiment, a processing method includes receiving an input of a medical condition or an observation about an infected animal; storing the input medical condition or the input observation as an item name in which distinction of completion and incompletion of treatment about the infected animal is managed; displaying whether the stored item name corresponds to either the completion or the incompletion of treatment in a visually recognizable manner in a follow-up examination about the infected animal; permitting an input of treatment information corresponding to the medical condition or the observation about the infected animal, when selection of a displayed item name has been made; and storing the input treatment information in association with the selected item name.

### BRIEF DESCRIPTION OF DRAWINGS

FIG. 1 is a function block diagram illustrating a configuration of an electronic medical record device according to the present embodiment;
FIG. 2 is a diagram illustrating an example of a data structure of an infected animal table;
FIG. 3 is a diagram illustrating an example of a data structure of a problem table;
FIG. 4 is a diagram illustrating an example of an infected animal selection screen;
FIG. 5 is a diagram (1) illustrating an example of a medical record information screen;
FIG. 6 is a diagram illustrating an example of an inquiry screen;
FIG. 7 is a diagram illustrating an example of a new examination screen;
FIG. 8 is a diagram illustrating an example of a reexamination screen;
FIG. 9 is a diagram illustrating a concept of processing in which a registration unit updates a problem table;
FIG. 10 is a diagram illustrating an example of a data structure of an updated problem table;
FIG. 11 is a diagram (2) illustrating an example of the medical record information screen;
FIG. 12 is a diagram (1) illustrating a screen example of when an interruption request has been received;
FIG. 13 is a diagram (2) illustrating the screen example of when an interruption request has been received;
FIG. 14 is a diagram (3) illustrating the screen example of when an interruption request has been received;
FIG. 15 is a diagram (4) illustrating the screen example of when an interruption request has been received;
FIG. 16 is a diagram illustrating a screen example of when examination completion has been received;
FIG. 17 is a flowchart illustrating a processing procedure of an electronic medical record device according to the present embodiment; and
FIG. 18 is a diagram illustrating a computer that executes a processing program.

### DESCRIPTION OF EMBODIMENTS

Preferred embodiments of the present invention will be explained with reference to accompanying drawings. Note that the invention is not limited by these embodiments.

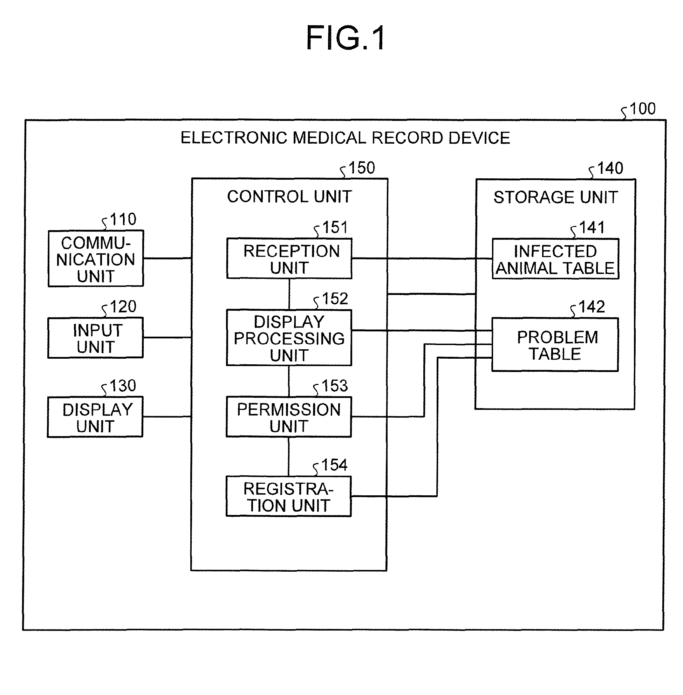

An example of a configuration of an electronic medical record device according to the present embodiment will be described. An electronic medical record device is an example of an infected animal electronic medical record device. FIG. 1 is a function block diagram illustrating a configuration of an electronic medical record device according to the present embodiment. As illustrated in FIG. 1, an electronic medical record device 100 includes a communication unit 110, an input unit 120, a display unit 130, a storage unit 140, and a control unit 150.

The communication unit 110 is a processing unit that is connected to a network by wire or wireless means, and performs data communication with other devices through the network. For example, the communication unit 110 is connected to a server that is a different device from an electronic medical record device through the network, and may receive information of an infected animal table 141 and a problem table 142 described below. The communication unit 110 corresponds to a communication device.

The input unit 120 is an input device that inputs various types of information. The input unit 120 corresponds to, for example, a keyboard, a mouse, a touch panel, or the like.

The display unit 130 is a display device that displays information output from the control unit 150. For example, the display unit 130 corresponds to a monitor, a liquid crystal display, a touch panel, or the like.

The storage unit 140 includes the infected animal table 141 and the problem table 142. The storage unit 140 corresponds to a random access memory (RAM), a read only memory (ROM), a semiconductor memory device such as a flash memory, or a storage device such as a hard disk drive (HDD).

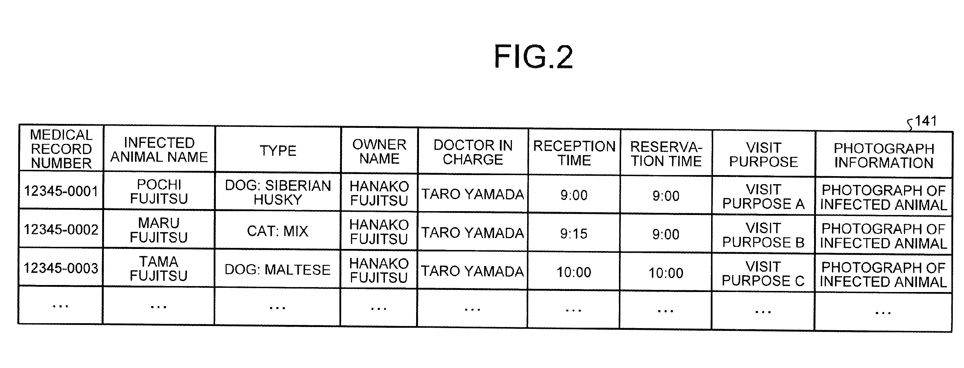

The infected animal table 141 is a storage area including items related to various types of information related to infected animals. In the following example, description will be given exemplarily illustrating a state in which the information is stored in a table format. How to store the information is not limited to the table format, and the information may be stored in any format as long as mutually associated pieces of information in the following description are stored in an associated state. FIG. 2 is a diagram illustrating an example of a data structure of an infected animal table. In the example of FIG. 2, the infected animal table 141 holds information regarding items of a medical record number, an infected animal name, a type, an owner name, a doctor in charge, a reception time, a reservation time, a visit purpose, and a photograph information in an associated state. One medical record number is given to one infected animal. That is, the infected animal and the medical record number are in one to one correspondence. It is desirable that at least information in which the medical record number and information that can identify an infected animal and the type of the infected animal as information of the infected animal are associated with each other is stored in the infected animal table 141. Further, items other than the above-described examples may also be held together.

The medical record number is information that uniquely identifies a medical record of an appropriate infected animal in a hospital. When a plurality of hospitals gathers and forms a group, the medical record number may be information uniquely identifiable in the plurality of hospitals that belongs to the same group. The infected animal name is information that indicates a name of an infected animal. The type is information that mainly indicates a type of an infected animal, including a type of an animal, such as "dog" or "cat," and a narrower type in the type of animal, such as "Siberian husky" in the "dog." The owner name is a name of an owner of an infected animal. The doctor in charge is information that indicates a doctor who takes care of an examination of an infected animal. The visit purpose is information that indicates a purpose of visit. The photograph information is photograph information of an infected animal.

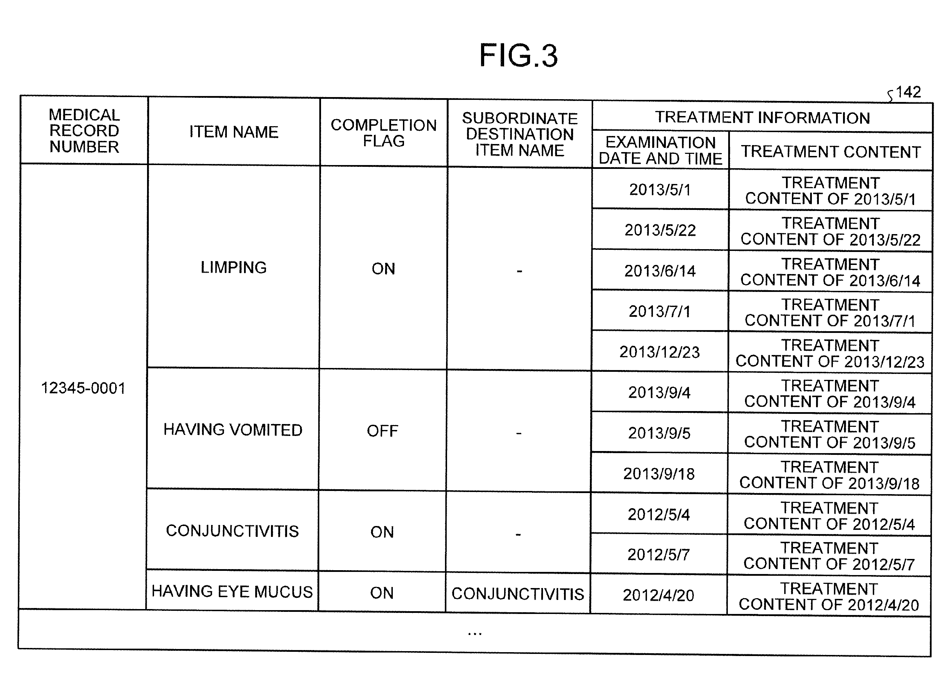

The problem table 142 is a storage area including items related to various types of information related to an examination, treatment content, and the like that have been performed for an infected animal. In the following example, description will be given by exemplarily illustrating a state in which the information is stored in a table format. How to store the information is not limited to the table format, and the information may be stored in any format as long as mutually associated pieces of information in the following description are stored in an associated state. FIG. 3 is a diagram illustrating an example of a data structure of the problem table. As illustrated in FIG. 3, the problem table 142 holds the medical record number, an item name, a completion flag, a subordinate destination item name, and treatment information in association with each other.

In FIG. 3, the medical record number is information that uniquely identifies a medical record of an appropriate infected animal. The item name is information that indicates a problem of the infected animal. A plurality of the same item names may be associated with one medical record number. Information stored in the problem is text data that can be input by the doctor who uses the present system, and may be information corresponding to a cause of disease of the infected animal, or may be information indicating a medical condition. The completion flag is information that indicates whether treatment of the infected animal regarding a problem has been completed. When the completion flag is "ON," the flag indicates that the treatment of the infected animal regarding the problem has been completed. For example, the completion of the treatment means a disease related to the problem of the infected animal has been cured. When the completion flag is "OFF," the flag indicates that the treatment of the infected animal regarding the problem has not been completed. For example, the incompletion of the treatment indicates that the disease related to the problem of the infected animal has not been cured.

In FIG. 3, the subordinate destination item name indicates an item name of a destination to which the item name is subordinate. For example, the subordinate destination item name corresponding to an item name "having eye mucus" is "conjunctivitis." Therefore, the item name "conjunctivitis" and the item name "having eye mucus" are associated with each other, and the subordinate destination item name indicates that the item name "having eye mucus" exists under the item name "conjunctivitis."

The treatment information includes information of an examination date and time and treatment content. The examination date and time indicates a date and time at which the doctor performed an examination for the infected animal. The treatment content includes, for example, information related to an examination result, such as an observation and a medical condition of when the doctor performed the examination to the infected animal, and the content of treatment that has been performed for the infected animal. By a combination of the medical record number, the item name, and the examination date and time, a record of one piece of treatment information can be identified.

Referring back to the description of FIG. 1. The control unit 150 includes a reception unit 151, a display processing unit 152, a permission unit 153, and a registration unit 154. The control unit 150 corresponds to, for example, an integrated device, such as an application specific integrated circuit (ASIC) or a field programmable gate array (FPGA). Further, the control unit 150 corresponds to, for example, an electronic circuit, such as a central processing unit (CPU) or a micro processing unit (MPU).

The reception unit 151 is a processing unit that receives an input of a medical condition or an observation about an infected animal, and registers the input medical condition or observation to the problem table 142 as an item name with which distinction between completion and incompletion of treatment about the infected animal is managed.

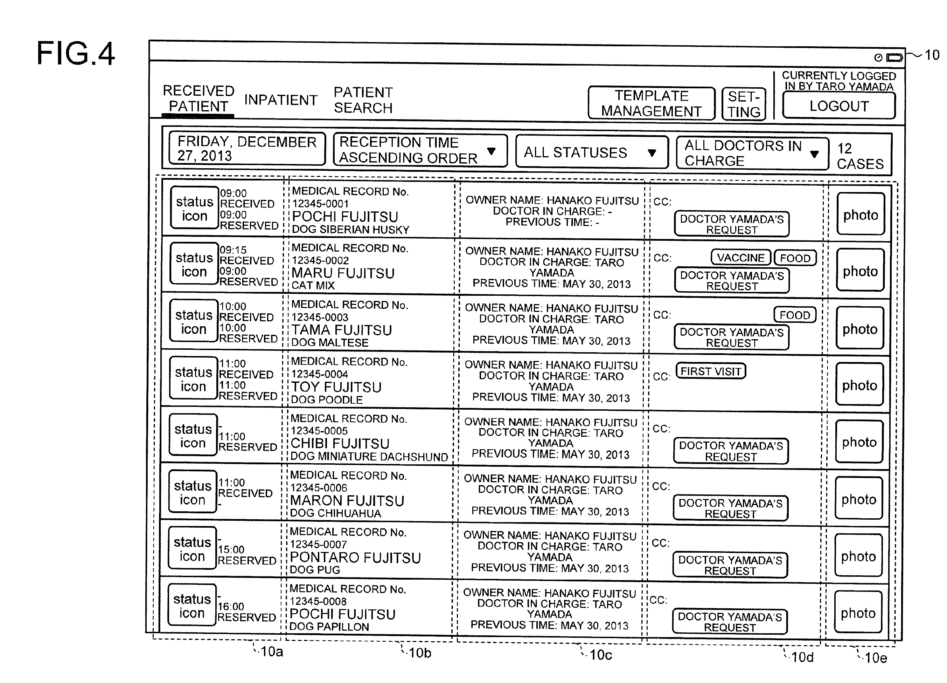

Here, an example of processing of the reception unit 151 will be described. When having received an input request from the input unit 120, the reception unit 151 generates an infected animal selection screen based on the infected animal table 141, and displays the infected animal selection screen in the display unit 130. FIG. 4 is a diagram illustrating an example of the infected animal selection screen. As illustrated in FIG. 4, an infected animal selection screen 10 includes a reception state 10a, first infected animal information 10b, second infected animal information 10c, a visit purpose 10d, and an infected animal photograph 10e.

For example, when generating the infected animal selection screen 10, the reception unit 151 generates information of the reception state 10a, the first infected animal information 10b, the second infected animal information 10c, the visit purpose 10d, and the infected animal photograph 10e for each record identified by the medical record number of the infected animal table 141.

The reception unit 151 sets the information of the reception time and the reservation time of the infected animal table 141 to the reception state 10a. The reception unit 151 sets the information of the medical record number, the infected animal name, and the type of the infected animal table 141 to the first infected animal information 10b. The reception unit 151 sets the information of the owner name and the doctor in charge of the infected animal table 141 to the second infected animal information 10c. The reception unit 151 sets the information of the visit purpose of the infected animal table 141 to the visit purpose 10d. For example, a "first examination" mark is displayed to an infected animal that first visits the hospital. The reception unit 151 sets the photograph information of the infected animal table 141 to the infected animal photograph 10e.

After displaying the infected animal selection screen 10 in the display unit 130, the reception unit 151 receives selection of an infected animal from the input unit 120. When having received the selection of an infected animal, the reception unit 151 identifies the medical record number of the selected infected animal, and outputs the information of the identified medical record number to the display processing unit 152. For example, in FIG. 4, when "Pochi Fujitsu" has been selected, the reception unit 151 outputs the medical record number "12345-0001" to the display processing unit 152.

The display processing unit 152 is a processing unit that displays whether the item name of the problem table 142 corresponds to either treatment completion or incompletion in the display unit 130 in a visually recognizable manner in a case of a follow-up examination of the infected animal.

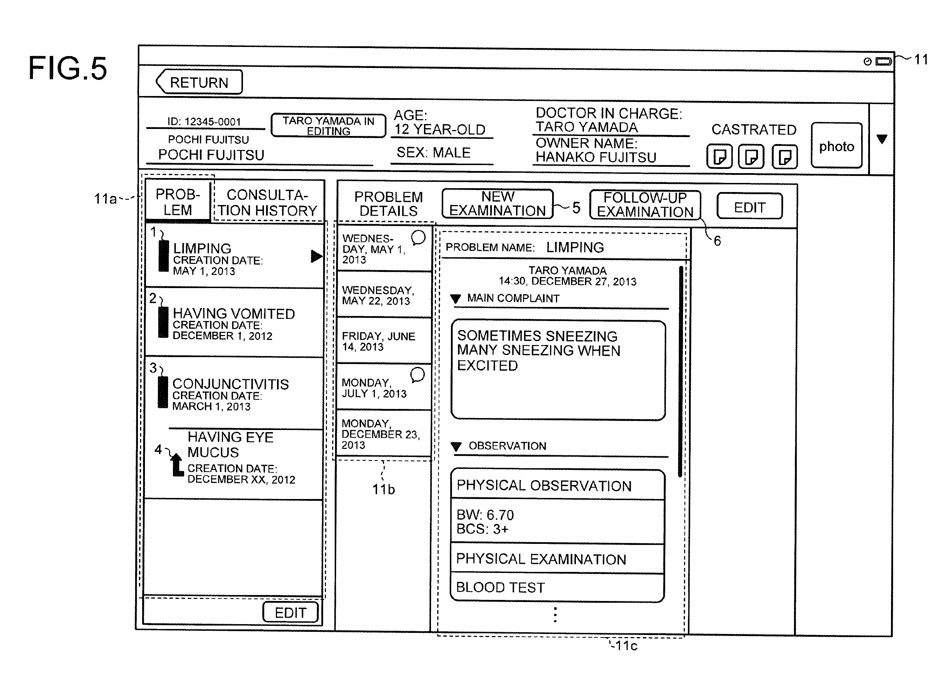

Here, an example of processing of the display processing unit 152 will be described. The display processing unit 152 acquires a medical record number from the reception unit 151, and identifies a record of the problem table 142 corresponding to the medical record number. The display processing unit 152 generates a medical record information screen based on the identified record, and displays the medical record information screen to the display unit 130. FIG. 5 is a diagram illustrating an example of the medical record information screen. As illustrated in FIG. 5, a medical record information screen 11 includes a problem display area 11a, an examination date and time display area 11b, and a treatment information display area 11c. The problem display area 11a, the examination date and time display area 11b, and the treatment information display area 11c will be described in order.

The problem display area 11a includes the item names included in the record of the problem table 142 corresponding to the medical record number acquired from the reception unit 151. For example, when having acquired the medical record number "12345-0001," the display processing unit 152 displays the item names included in the record of the medical record number "12345-0001" in the problem display area 11a. For example, the item names included in the record of the medical record number "12345-0001" are "limping," "having vomited," "conjunctivitis," and "having eye mucus."

When displaying each item name in the problem display area 11a, the display processing unit 152 displays the item names in a hierarchal manner based on the subordinate destination item names of the problem table 142. For example, the subordinate destination item name of the item name "having eye mucus" is "conjunctivitis." Therefore, the display processing unit 152 displays the item name "conjunctivitis" and the item name "having eye mucus" such that these item names being in a hierarchal relationship can be recognized. Further, here, the display processing unit 152 may display the item names such that the item name "conjunctivitis" being in a higher rank in the hierarchy in the hierarchal relationship can be recognized. Note that the display processing unit 152 arranges an item name that does not have the subordinate destination item name in a highest rank in the hierarchy.

Further, when displaying each item name in the problem display area 11a, the display processing unit 152 generates a screen and displays whether treatment of the problem corresponding to the item name has been completed in a visually recognizable manner based on the completion flag. For example, when the completion flag is "ON" and the treatment of the problem corresponding to the item name has been completed, the display processing unit 152 sets a color of characters of the item name or of a mark of the item name to a first color. For example, when the completion flag of the item name "limping" is "ON," the display processing unit 152 sets the color of a mark 1 to the first color.

In contrast, when the completion flag is "OFF" and the treatment of the problem corresponding to the item name has not been completed, the display processing unit 152 sets the color of the characters of the item name or of the mark of the item name to a second color. The second color may be any color as long as the color is different from the first color. For example, when the completion flag of the item name "having vomited" is "OFF," the display processing unit 152 sets the color of a mark 2 to the second color. Note that the display processing unit 152 may output the completed problem and the incomplete problem with a distinguishing way other than the color. For example, between the completion and the incompletion, the display processing unit 152 may change the shape of a frame of a display region or a font of each problem. Alternatively, the display processing unit 152 may display a mark indicating the completion or the incompletion in the display region of each problem.

The examination date and time display area 11b includes an examination date and time corresponding to the item name selected through the input unit 210. For example, when the item name "limping" has been selected, the display processing unit 152 displays the examination date and time corresponding to the item name "limping" of the problem table 142.

The treatment information display area 11c includes the information of the treatment content corresponding to the examination date and time selected through the input unit 120. For example, when an examination date and time "May 1, 2013" of the item name "limping" has been selected, the display processing unit 152 displays the information of the treatment content corresponding to the examination date and time "May 1, 2013" of the item name "limping."

By reference to the medical record information screen 11 illustrated in FIG. 5, the doctor can easily recognize information of what kind of problem an animal to be examined suffered from in the past and whether treatment related to the problem has been completed, and a history and a course of treatment.

The display processing unit 152 receives either a "new examination" or a "follow-up examination" after displaying the medical record information screen 11. For example, the display processing unit 152 receives a new examination when a new examination button 5 has been pressed through the input unit 120, and receives a follow-up examination when a follow-up examination button 6 has been pressed. A case of having received the new examination and a case of having received the follow-up examination will be described in order.

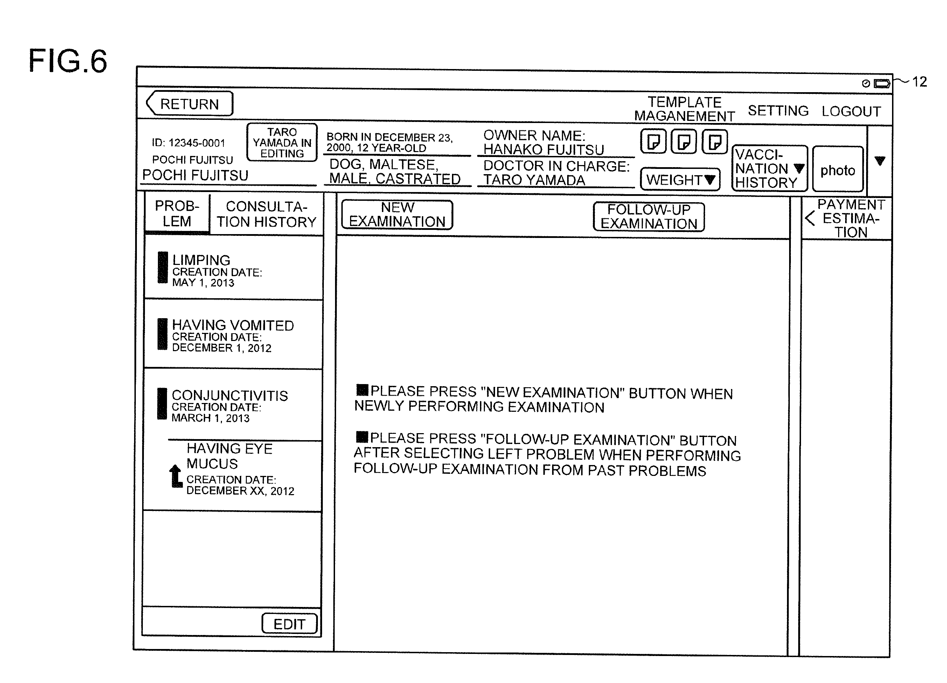

Note that the display processing unit 152 may display an inquiry screen 12 as illustrated in FIG. 6, and may allow "new examination" or "follow-up examination" to be selected. FIG. 6 is a diagram illustrating an example of the inquiry screen. For example, it can be considered that the display processing unit 152 outputs the inquiry screen 12 when an examination of one infected animal has been completed and before or when an examination of a next infected animal is started. In the inquiry screen 12, when a problem has been selected and the "follow-up examination" button has been pressed, the display processing unit 152 outputs a reexamination screen about the selected problem.

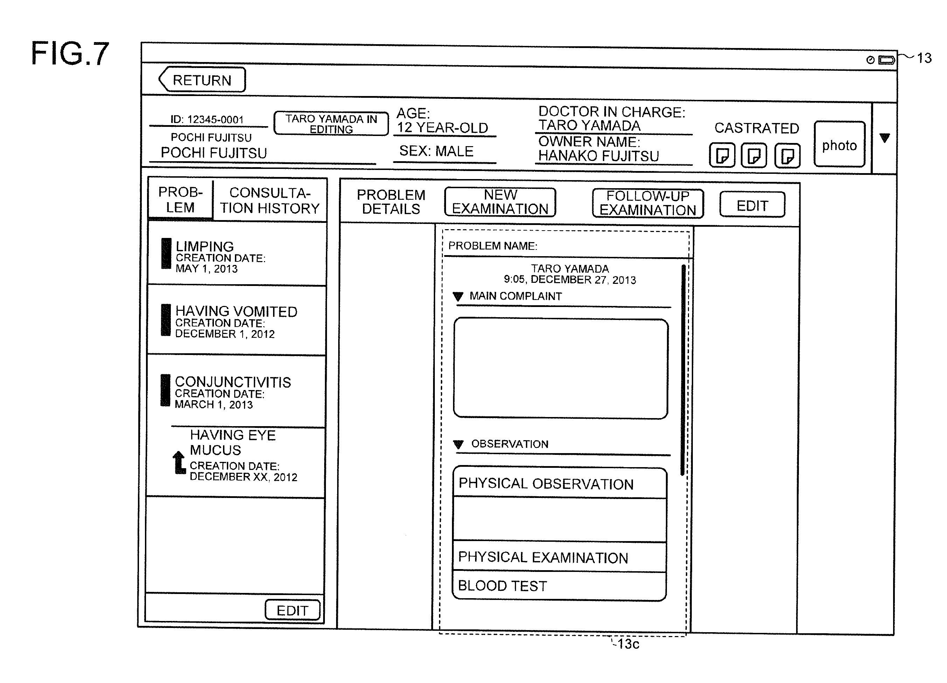

Processing of when the display processing unit 152 has received a new examination will be described. When having received a new examination, the display processing unit 152 generates a new examination screen, and displays the new examination screen in the display unit 130. FIG. 7 is a diagram illustrating an example of the new examination screen. For example, the doctor operates the input unit 120 to input various types of treatment information in a treatment information display area 13c in a new examination screen 13. For example, the doctor inputs treatment information, such as a problem name, a main complaint, an observation, and a medical condition, and an examination date and time. Regarding the information of an examination date and time, if the display processing unit 152 acquires current time information, the display processing unit 152 can display the acquired current time. The doctor may overwrite and modify displayed information of the examination date and time. The information input to the treatment information display area 13c is registered to the problem table 142 by the registration unit 154 described below.

Processing of when the display processing unit 152 has received a follow-up examination will be described. As the premise of receiving the follow-up examination, here, one of the problems in the problem display area 11a has been selected. For example, when the follow-up examination button 6 has been pressed in a state where the problem of "limping" in the problem display area 11a had been selected, a follow-up examination related to the problem of "limping" will be made.

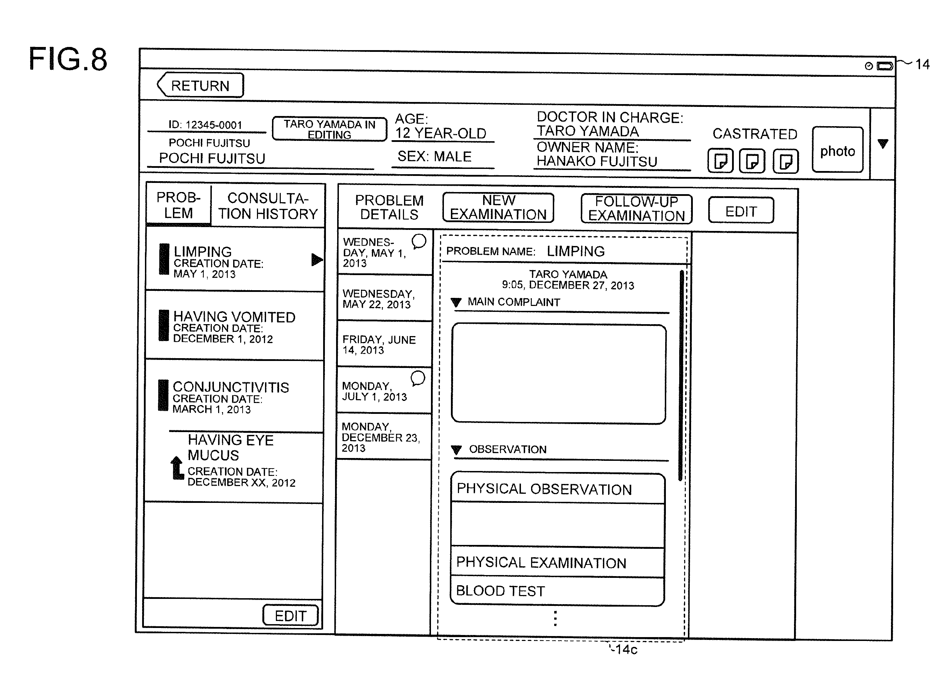

When the follow-up examination button 6 has been pressed, the display processing unit 152 displays the reexamination screen in the display unit 130. Note that, here, the display processing unit 152 confirms whether treatment of an item name that has been selected before the follow-up examination button 6 is pressed has been completed, that is, whether the completion flag corresponding to the item name in the problem table 142 corresponding to the medical record number of an infected animal to be examined is "ON" or "OFF." When the treatment is incomplete, that is the completion flag is "OFF," the display processing unit 152 outputs a reexamination screen 14 illustrated in FIG. 8. Further, when the treatment has been completed, that is, the completion flag is "ON," the display processing unit 152 outputs a message screen that prompts reselection of an item name, the treatment is incomplete. Alternatively, when the treatment has been completed, that is, the completion flag is "ON," the display processing unit 152 outputs a screen that inquires whether changing the selected item name from the treatment completion to the state of incompletion. When having received an input of changing the item name to the state of incompletion, the display processing unit 152 changes the completion flag of the appropriate item name to "OFF," and outputs the reexamination screen 14 illustrated in FIG. 8. FIG. 8 is a diagram illustrating an example of the reexamination screen. Assume that the reexamination screen 14 illustrated in FIG. 8 is a screen of when a follow-up examination of the problem "limping" is performed. The doctor operates the input unit 120 to input the treatment information, such as a main complaint, an observation, and a medical condition, in a treatment information display area 14c. The examination date and time may also be an object to be input. If the display processing unit 152 receives the current time information, the display processing unit 152 may display the acquired current time without having the examination date and time as the input item. The information input to the treatment information display area 14c is registered in the problem table 142 by the registration unit 154 described below.

Referring back to the description of FIG. 1. The permission unit 153 is a processing unit that determines whether permitting an input of the treatment information corresponding to the medical condition or the observation about the infected animal when a displayed item name is selected, on the reexamination screen. The permission unit 153 outputs a determination result to the registration unit 154.

For example, the permission unit 153 compares the selected item name and a problem table 142 corresponding to the medical record number, and permits an input of the treatment information when the completion flag of the item name is "OFF." In contrast, when the completion flag of the item name is "ON," the permission unit 153 does not permit the input of the treatment information.

Note that the permission unit 153 does not determine whether permitting an input regarding the information input to the treatment information display area 13c of the new examination screen 13.

The registration unit 154 is a processing unit that registers the treatment information permitted by the permission unit 153 on the reexamination screen to the problem table 142. Further, the registration unit 154 registers the information input to the treatment information display area 13c of the new examination screen 13 to the problem table 142.

An example of when the registration unit 154 processes information input to a new examination screen will be described. For example, the registration unit 154 registers the information input to the treatment information display area 13c illustrated in FIG. 7 to the problem table 142 in association with the medical record number. The registration unit 154 additionally registers the information input to the problem name of the treatment information display area 13c as new data of a value of the column of the item name to the problem table 142. Further, the registration unit 154 acquires the information of whether treatment of an appropriate problem has been completed through the input unit 120, and reflects the information to the completion flag. For example, when having acquired the information indicating the treatment has been completed, the registration unit 154 sets the completion flag to "ON." In contrast, when having acquired the information indicating the treatment has not been completed, the registration unit 154 sets the completion flag to "OFF."

An example of when the registration unit 154 processes information input to a reexamination screen will be described. For example, when an input of the information input to the treatment information display area 14c illustrated in FIG. 8 has been permitted, the registration unit 154 additionally registers the information of the examination date and time input to or displayed in the treatment information display area 14c to the problem table 142 as new data of a value of the column of the examination date and time in association with the item name of an appropriate medical record number.

For example, assume that an input of the information input to the treatment information display area 14c has been permitted by the permission unit 153, on the reexamination screen where the problem "limping" illustrated in FIG. 8 has been selected. In this case, the registration unit 154 additionally registers the information of the treatment information display area 14c to the treatment information of the problem table 142, in association with the item name "limping" of the medical record number "12345-0001" of the problem table 142.

Next, another processing of the registration unit 154 will be described. Hereinafter, processing of when the registration unit 154 has received change of a problem name, processing of when the registration unit 154 has received an interruption request, and processing of when the registration unit 154 has received diagnosis completion will be described.

Processing of when the registration unit 154 has received change of a problem name will be described. For example, when the doctor examines an infected animal, a cause of disease that has not been clear so far may sometimes become clear. In such a case, if the problem name set at the timing when the cause of disease was unclear can be changed to the problem name that indicates the cause of disease, the problem can be more easily identified when the problem is selected later. For example, with respect to the medical condition of "having vomited" set as the problem name before, there is a case in which the cause of disease of having vomited being "enteritis" becomes clear as a result of an examination. Therefore, the electronic medical record device 100 receives change of the problem name of the treatment information display area 14c on the reexamination screen by an operation of the input unit 120 by the doctor. For example, in the above example, the electronic medical record device 100 changes the problem name of the treatment information display area 14c from the "having vomited" to the "enteritis."

When the problem name of the treatment information display area 14c on the reexamination screen 14 has been changed, the registration unit 154 updates the problem table 142 such that information related to the changed problem and the information related to the problem before change mutually have a hierarchal relationship. Further, here, the registration unit 154 may update the problem table 142 such that the information related to the problem before change is arranged at a lower rank of the hierarchal relationship than the information related to the changed problem.

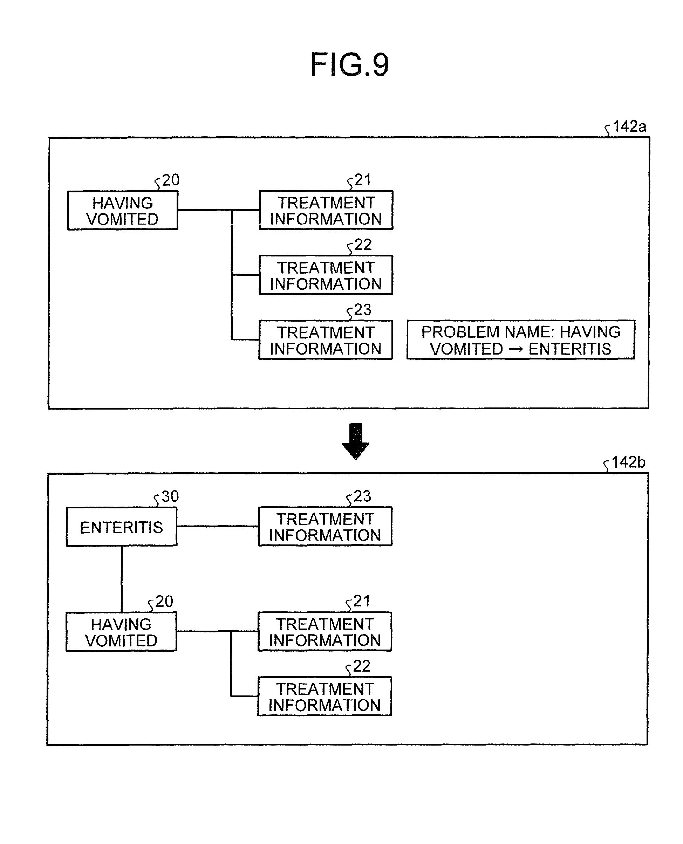

FIG. 9 is a diagram illustrating a concept of processing in which the registration unit updates the problem table. In FIG. 9, a relationship of data stored in the problem table 142 before the update is expressed as a problem table 142a, and a relationship of data stored in the updated problem table 142 after the update is expressed as a problem table 142b. For example, the problem table 142a illustrates, as illustrated in the problem table 142 of FIG. 3, a state in which pieces of treatment information 21 to 23 respectively input in examinations of three times where the examination dates and times are "2013/9/4," "2013/9/5," and "2013/9/18" are associated with the item name "having vomited" 20. That is, the problem table 142a illustrates a state in which after the problem "having vomited" 20 was selected and the input of the follow-up examination was received on September 18, 2013, in a state where the treatment information 21 corresponding to "2013/9/4" and the treatment information 22 corresponding to "2013/9/5" had been stored, but change of the problem name has not been made. Assume that, from this state, the problem name of the treatment information 23 has been changed from the "having vomited" to the "enteritis" at the time of an examination in which an input of the treatment information 23 is performed.

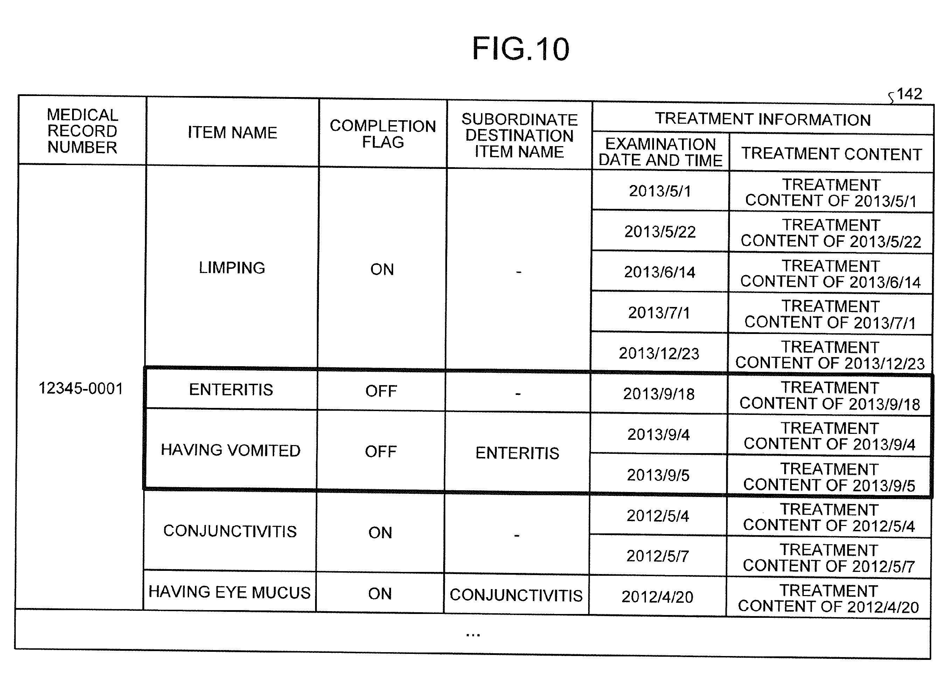

In this case, the registration unit 154 generates data of the item name "enteritis" 30 as a new problem, in place of the treatment information 23 temporarily registered as narrow data of the problem "having vomited" 20. The registration unit 154 then associates the treatment information 23 with the problem of the item name "enteritis" 30. The registration unit 154 then sets a hierarchal relationship between the item name "enteritis" 30 and the item name "having vomited" 20, and associates the item name "having vomited" 20 with a lower rank of the item name "enteritis" 30. Correspondence between the item name 20 and the treatment information 21 and 22 is maintained. For example, the problem table 142 illustrated in FIG. 3 is updated to a problem table illustrated in FIG. 10. FIG. 10 is a diagram illustrating an example of a data structure of an updated problem table. As illustrated in FIG. 10, the registration unit 154 newly adds a record related to the item name "enteritis", and sets a subordinate destination item name of the item name "having vomited" to the "enteritis."

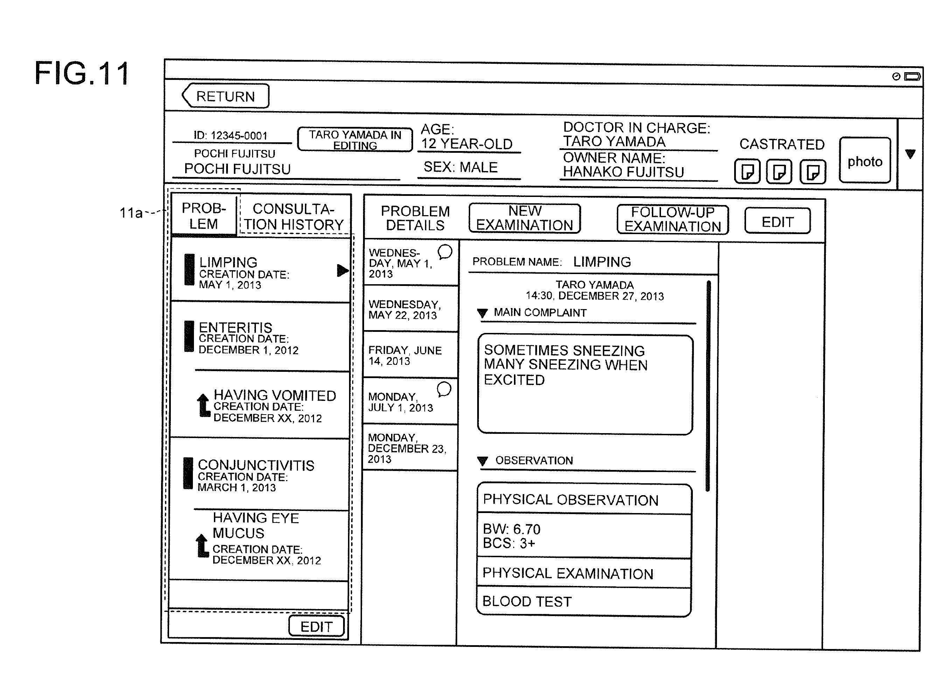

For example, as illustrated in FIG. 10, when the problem table 142 has been updated, the medical record information screen displayed in the display unit 130 by the display processing unit 152 is changed to one illustrated in FIG. 11. FIG. 11 is a diagram (2) illustrating an example of the medical record information screen. As illustrated in FIG. 11, the "having vomited" is displayed under the item name "enteritis" of the problem display area 11a such that the both problems are adjacent to each other.

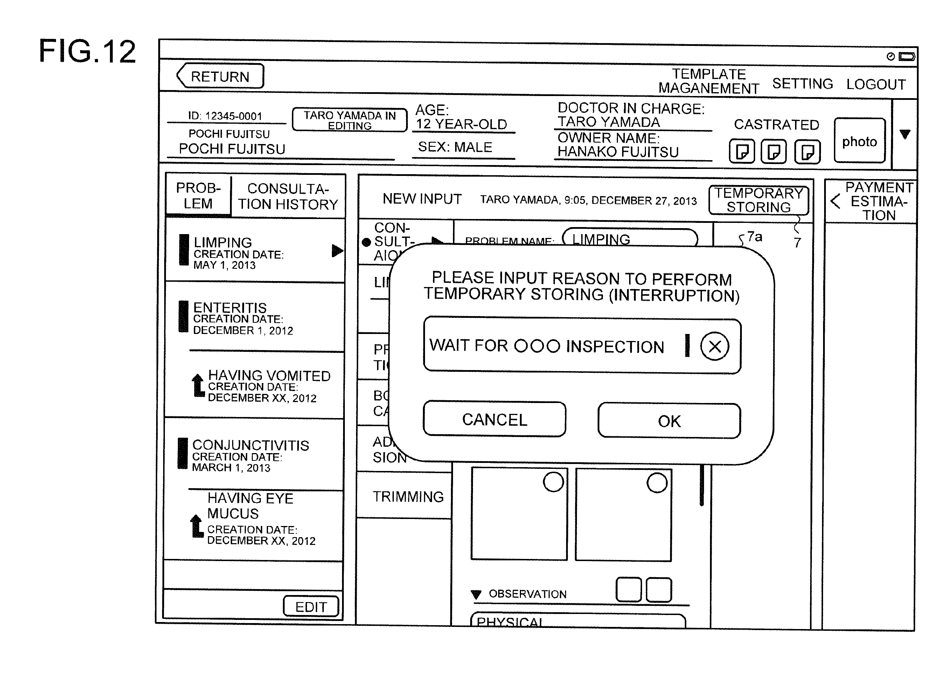

Next, processing of when the registration unit 154 has received an interruption request will be described. FIGS. 12 to 15 are diagrams illustrating a screen example of when the registration unit 154 has received an interruption request. For example, there is a case in which an inspection is performed during an examination of a certain infected animal, and the examination of the infected animal is interrupted and an examination of another infected animal is performed until an inspection result is obtained. When performing such interruption of an examination, the doctor operates the input unit 120 to press a temporary storing button 7 illustrated in FIG. 12. When the temporary storing button 7 is pressed, the registration unit 154 displays a window 7a, and receives an input of a reason of temporary storing. For example, the registration unit 154 registers the reason of temporary storing and the medical record number, an examination of which has been interrupted, in the problem table 142 in association with each other, and performs a display request of the infected animal selection screen to the reception unit 151. Further, the registration unit 154 registers the information registered in the treatment information display area 11c to the problem table 142.

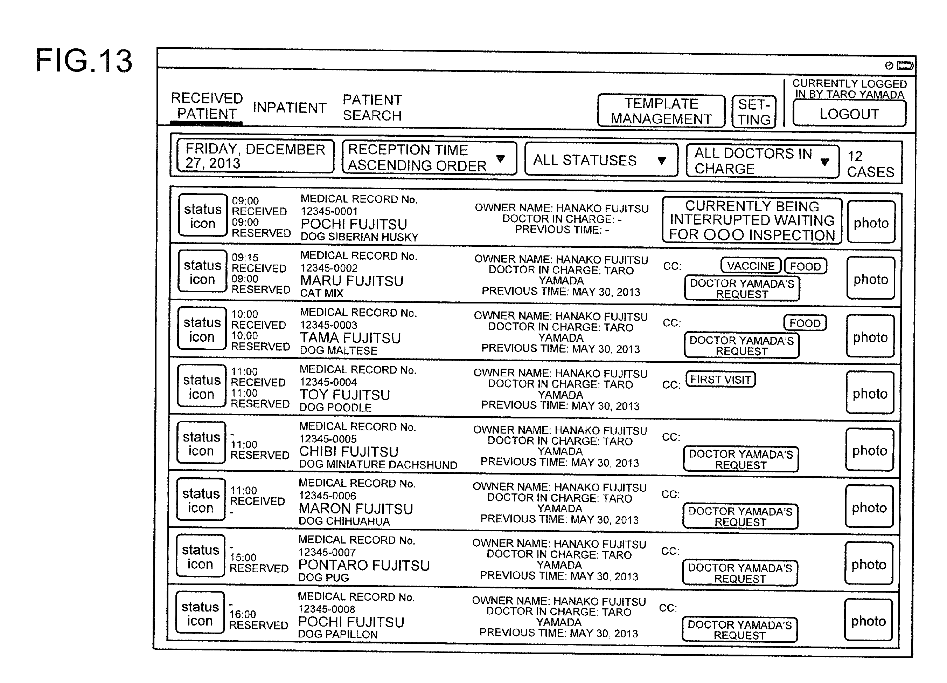

When having received a display request of the infected animal selection screen after the processing of interruption has been made, the reception unit 151 displays the infected animal selection screen illustrated in FIG. 13. When the reason of temporary storing is associated with the medical record number, the reception unit 151 displays the reason of temporary storing in an appropriate record. In the example of FIG. 13, the processing related to the medical record number "12345-0001" is temporarily stored, and the reason of temporary storing is "currently being interrupted for waiting for ooo inspection".

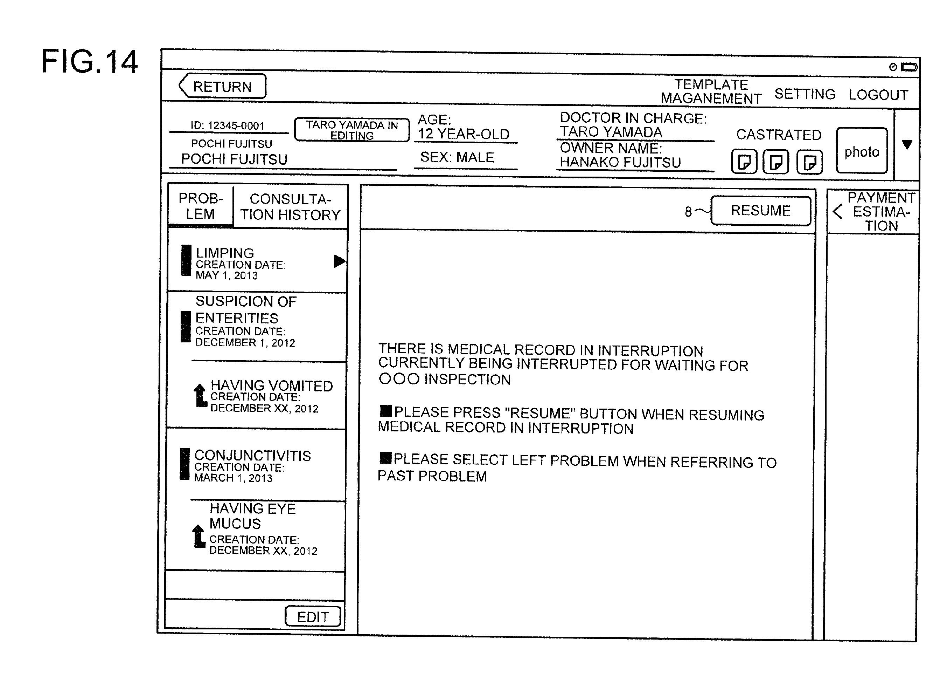

When resuming the examination, that is, when resuming a data input regarding the temporarily stored problem, the doctor operates the input unit 120 to select the temporarily stored record of FIG. 13. When the temporarily stored record has been selected, the reception unit 151 displays the screen illustrated in FIG. 14, and the doctor operates the input unit 120 to press a resume button 8.

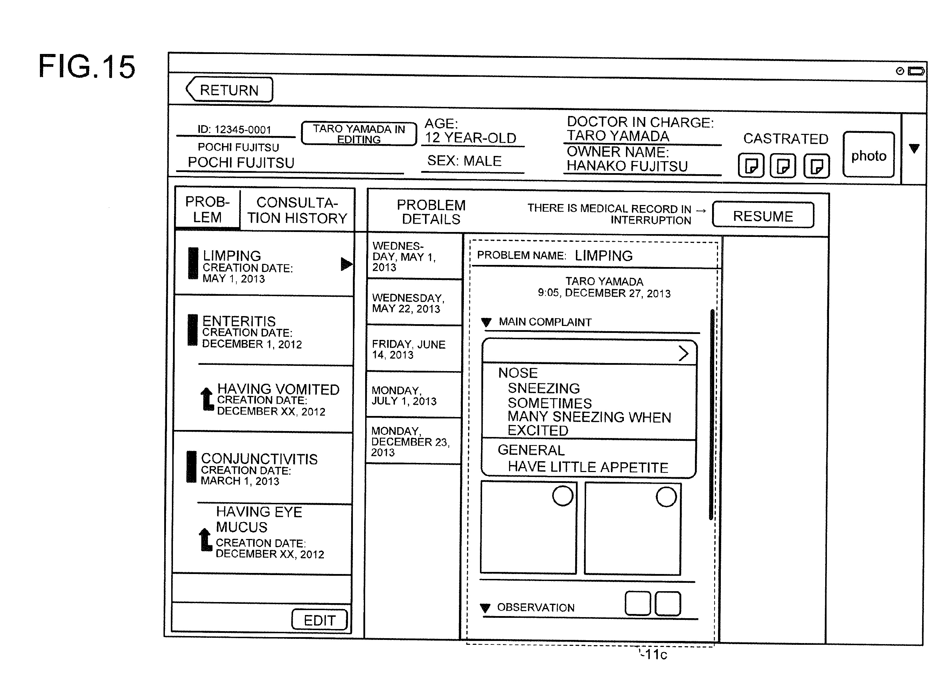

When the resume button 8 of FIG. 14 has been pressed, the registration unit 154 reads out the temporarily stored treatment information from the problem table 142 using the medical record number selected in FIG. 13 as a key, and displays the treatment information in the treatment information display area 11c, thereby displaying the screen illustrated in FIG. 15. For example, in the treatment information display area 11c, information input to the treatment information display area 11c until the temporary storing is performed.

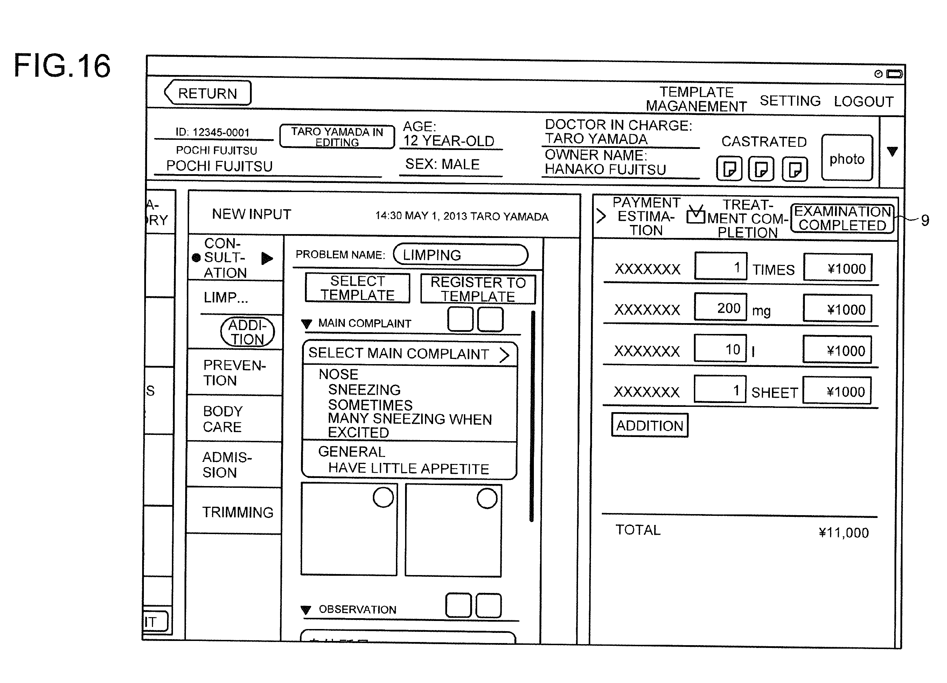

Next, processing of when the registration unit 154 has received diagnosis completion. FIG. 16 is a diagram illustrating a screen example of when the registration unit 154 has received diagnosis completion. As illustrated in FIG. 16, when having completed an examination, the doctor presses an examination completion button 9 of FIG. 16. When the examination completion of FIG. 16 has been pressed, the registration unit 154 performs predetermined payment estimation, and output an estimation result. Note that the doctor may input information of whether treatment of the infected animal related to the problem has been completed at a timing when pressing the examination completion button 9 of FIG. 16. For example, it can be considered to input the information of completion by inputting of ON to the treatment completion flag. The registration unit 154 sets the completion flag of an appropriate record of the problem table 142 to ON or OFF according to an input result of completion or incompletion of the treatment.

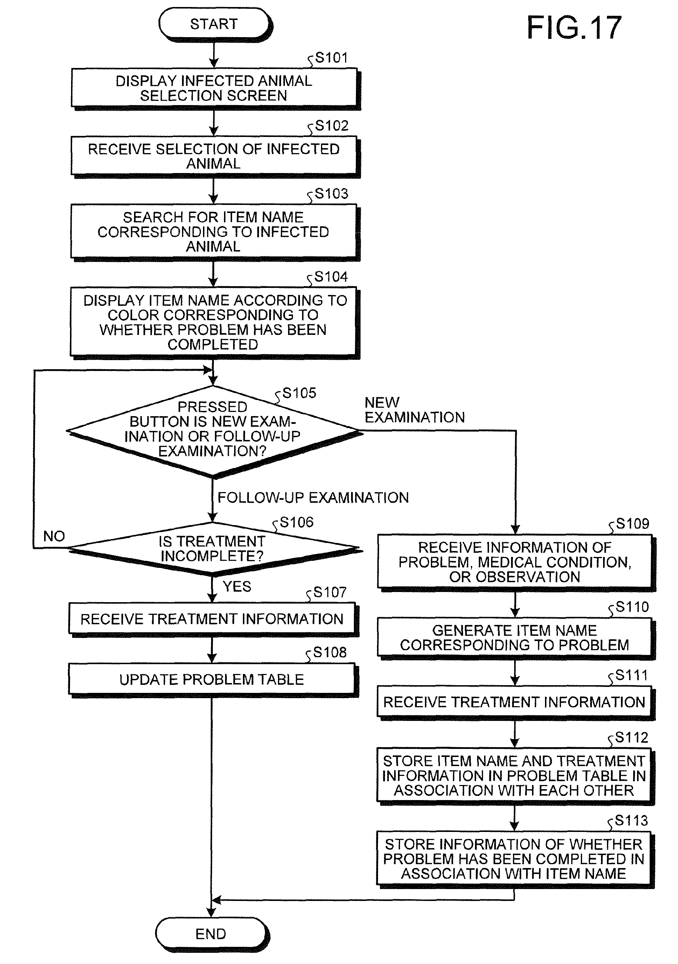

Next, a processing procedure of the electronic medical record device 100 according to the present embodiment will be described. FIG. 17 is a flowchart illustrating a processing procedure of an electronic medical record device according to the present embodiment. The electronic medical record device 100 executes processing illustrated in FIG. 17 upon receiving an input request from the input unit 120, for example.

As illustrated in FIG. 17, the electronic medical record device 100 displays the infected animal selection screen (step S101), and receives selection of an infected animal (step S102). The electronic medical record device 100 searches for an item name corresponding to the selected infected animal (step S103), determines a display state including a display color of the item name according to whether the problem has been completed, and generates and displays a screen according to the determination (step S104). For example, in step S104, the electronic medical record device 100 sets the item name to the first color when the completion flag is ON, and sets the item name to the second color when the completion flag is OFF. Further, in step S104, problems can be displayed in a list, and at that time, the problems may be displayed in a state in which mutually associated problems are arranged adjacent to each other.

The electronic medical record device 100 receives selection of an item name (step S106). Then, the electronic medical record device 100 determines whether having received an input of either a new examination or a follow-up examination (step S105). In step S105, the electronic medical record device 100 may determine whether the input is a follow-up examination according to whether the follow-up examination button has been pressed in the medical record information screen 11.

When the input is a follow-up examination of a problem (the follow-up examination of step S105), the electronic medical record device 100 determines whether treatment of the problem of the item name selected in S106 is incomplete (step S106). That is, the electronic medical record device 100 determines whether the completion flag of the problem of the selected item name is "OFF" in the problem table 142. When the treatment is incomplete, that is, the completion flag is "OFF" (Yes in step S106), the electronic medical record device 100 receives the treatment information (step S107), and updates the problem table 142 (step S108). Note that, in step S108, the electronic medical record device 100 receives the information of whether the problem has been completed, and stores the information of whether the problem has been completed and the item name in the problem table 142 in association with each other. Further, in step S106, when the treatment has been completed, that is the completion flag is "ON" (No in step S106), the electronic medical record device 100 returns to step S106 so as to prompt selection of an item name of incompletion. Alternatively, it can be considered that the electronic medical record device 100 does not return to step S106, and outputs a screen that inquires whether changing the selected item name, the treatment of which has been completed, to treatment incompletion. When having received an input of changing the item name to the treatment incompletion, the electronic medical record device 100 changes the completion flag of an appropriate item name in the problem table 142 from "ON" to "OFF," and proceeds to step S107.

Meanwhile, when the input is a new examination of a problem (the new examination of step S105), the electronic medical record device 100 receives the problem and the information of the medical condition or the observation (step S109). The electronic medical record device 100 generates an item name corresponding to the problem (step S110), and receives the treatment information (step S111).

The electronic medical record device 100 stores the item name and the treatment information in the problem table in association with each other (step S112), and stores the information of whether the problem has been completed and the item name in association with each other (step S113).

Next, effects of the electronic medical record device 100 according to the present embodiment will be described. The electronic medical record device 100 stores the medical condition or the observation about an infected animal as the item name, and stores the information indicating completion or incompletion of treatment in association with the item name. Therefore, in the case of a follow-up examination, the electronic medical record device 100 can generate and display a display screen with which whether the item name corresponds to either the completion or incompletion of treatment can be visually recognized. When selection of the item name has been made, the electronic medical record device 100 permits an input of the treatment information corresponding to the medical condition or the observation about the infected animal, and stores the input treatment information in association with the selected item name. Therefore, according to the electronic medical record device 100, the doctor can easily recognize the problem that is an object to be input of the treatment information.

Further, according to the electronic medical record device 100, an object item name, an input of the treatment information of which is to be permitted, is an item name, treatment of which is incomplete, and does not include an item name, treatment of which has been completed. Therefore, the doctor can be prevented from inputting wrong treatment information related to a problem, treatment of which has been completed.

Further, according to the electronic medical record device 100, when designation of an item name different from the item name that is currently being input, in addition to the input of the treatment information, the electronic medical record device 100 generates and displays a screen in a state where the item name and the different item name are associated with each other. Accordingly, past diagnoses and medical conditions up to the condition of a disease indicated by the different item name can be displayed in an associated state, and thus how the condition of a disease gets to the current condition can be easily known at a glance.

Further, according to the electronic medical record device 100, the electronic medical record device 100 generates and displays a screen in a state where the different item name has a hierarchal relationship with the item name. At this time, a screen on which the different item name is a higher rank item than the item name can be generated and displayed. Therefore, the doctor can easily recognize the latest medical condition.

Further, according to the electronic medical record device 100, when a plurality of item names is displayed, the electronic medical record device 100 can generate and display a screen on which associated item names are arranged in adjacent positions. Therefore, the doctor can easily recognize the past medical conditions and diagnoses and the latest diagnosis.

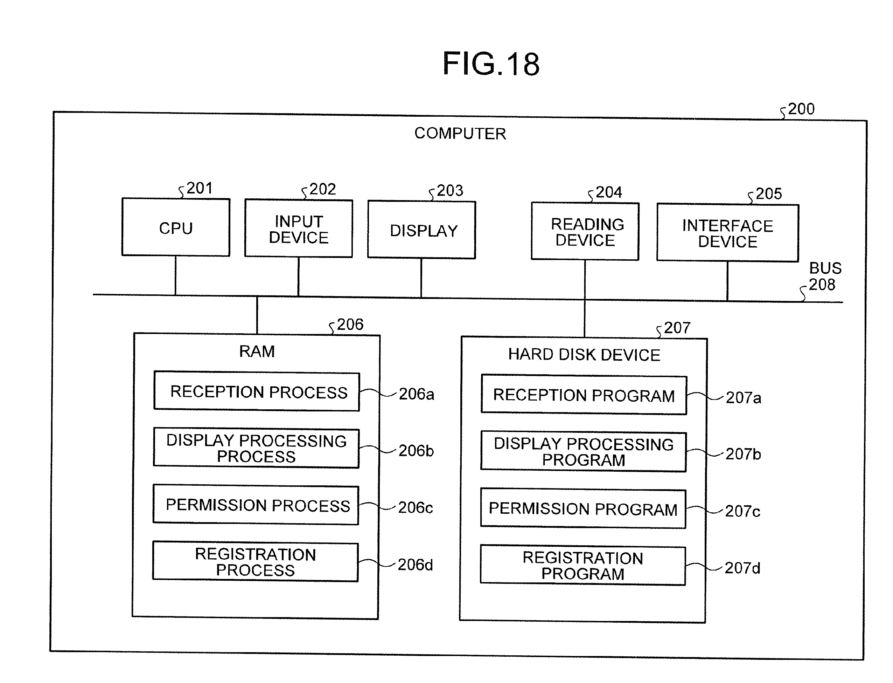

Next, an example of a computer that executes a processing program that realizes a function similar to the electronic medical record device 100 illustrated in the above-described embodiment will be described. FIG. 18 is a diagram illustrating an example of a computer that executes a processing program.

As illustrated in FIG. 18, a computer 200 includes a CPU 201 that executes various types of arithmetic processing, an input device 202 that receives an input of data from a user, and a display 203. Further, the computer 200 includes a reading device 204 that reads out a program and the like from a storage medium, and an interface device 205 that gives and receives data to/from other computers through a network. Further, the computer 200 includes a RAM 206 that temporarily stores various types of information, and a hard disk device 207. Then, the devices 201 to 207 are connected to a bus 208.

The hard disk device 207 includes a reception program 207a, a display processing program 207b, a permission program 207c, and a registration program 207d. The CPU 201 reads out the programs 207a to 207d, and develops the programs in the RAM 206.

The reception program 207a functions as a reception process 206a. The display processing program 207b functions as a display processing process 206b. The permission program 207c functions as a permission process 206c. The registration program 207d functions as a registration process 206d.

For example, the reception process 206a corresponds to the reception unit 151. The display processing process 206b corresponds to the display processing unit 152. The permission process 206c corresponds to the permission unit 153. The registration process 206d corresponds to the registration unit 154.

Note that the programs 207a to 207d are not necessarily be stored in the hard disk device 207 from the beginning. For example, the programs are stored in a "portable physical medium," such as a flexible disk (FD), a CD-ROM, a DVD disk, a magneto-optical disk, or an IC card, which is inserted to the computer 200. Then, the computer 200 reads out the programs 207a to 207d from the portable physical medium and executes the programs 207a to 207d.

According to an embodiment of the present invention, an effect to generate and display a screen that enables a user to easily recognize a problem that is an object to be input of treatment information is exerted.

## Claims

1. A processing method executed by a computer, the processing method comprising:
receiving an input of a medical condition or an observation about an infected animal;
storing the input medical condition or the input observation as an item name in which distinction of completion and incompletion of treatment about the infected animal is managed;
displaying whether the stored item name corresponds to either the completion or the incompletion of treatment in a visually recognizable manner in a follow-up examination about the infected animal;
permitting an input of treatment information corresponding to the medical condition or the observation about the infected animal, when selection of a displayed item name has been made; and
storing the input treatment information in association with the selected item name.

2. The processing method according to claim 1, wherein an object item name, an input of the treatment information of which is permitted, has incomplete treatment, and does not include an item name, treatment of which has been completed.

3. The processing method according to claim 1, wherein the displaying displays the item name and the different item name in association with each other, when designation of a different item name from the selected item name is further received, in addition to the input of the treatment information.

4. The processing method according to claim 3, wherein the displaying displays the differed item name as an item having a hierarchal relationship with the item name.

5. The processing method according to claim 3, wherein, the displaying displays the item name and the different item name associated with each other in adjacent positions, when a plurality of item names is displayed.

6. A process program including:
receiving an input of a medical condition or an observation about an infected animal;
storing the input medical condition or the input observation as an item name in which distinction of completion and incompletion of treatment about the infected animal is managed;
displaying whether the stored item name corresponds to either the completion or the incompletion of treatment in a visually recognizable manner in a follow-up examination about the infected animal;
permitting an input of treatment information corresponding to the medical condition or the observation about the infected animal, when selection of a displayed item name has been made; and
storing the input treatment information in association with the selected item name.

7. The process program according to claim 6, wherein an object item name, an input of the treatment information of which is permitted, has incomplete treatment, and does not include an item name, treatment of which has been completed.

8. The process program according to claim 6, wherein, the displaying displays the item name and the different item name in association with each other, when designation of a different item name from the selected item name is further received, in addition to the input of the treatment information.

9. The process program according to claim 8, wherein the displaying displays the differed item name as an item having a hierarchal relationship with the item name.

10. The process program according to claim 8, wherein, the displaying displays the item name and the different item name associated with each other in adjacent positions, when a plurality of item names is displayed.

11. An infected animal electronic medical record device (100) comprising:
a reception unit(151) that receives an input of a medical condition or an observation about an infected animal, and stores the input medical condition or the input observation as an item name in which distinction of completion and incompletion of treatment about the infected animal is managed in a storage unit;
a display processing unit(152) that displays whether the stored item name corresponds to either the completion or the incompletion of treatment in a visually recognizable manner in a follow-up examination about the infected animal;
a permission unit(153) that, when selection of a displayed item name has been made, permits an input of treatment information corresponding to the medical condition or the observation about the infected animal; and
a registration unit(154) that stores the input treatment information in association with the selected item name in the storage unit.

12. The infected animal electronic medical record device(100) according to claim 11, wherein an object item name, an input of the treatment information of which is permitted by the permission unit, has incomplete treatment, and does not include an item name, treatment of which has been completed.

13. The infected animal electronic medical record device(100) according to claim 11, wherein, when the display processing unit(152) displays the item name and the different item name in association with each other, when designation of a different item name from the selected item name is further received, in addition to the input of the treatment information.

14. The infected animal electronic medical record device according to claim 13, wherein the display processing unit(152) displays the different item name as an item having a hierarchal relationship with the item name.

15. The infected animal electronic medical record device according to claim 13, wherein, the display processing unit(152) displays the item name and the different item name associated with each other to be arranged in adjacent positions, when a plurality of item names is displayed.
